# EUROPEAN PATENT APPLICATION

(11) **EP 3 825 704 A1**
(43) Date of publication of application: **26.05.2021**
(21) Application number: 19837341.7
(22) Date of filing: 19.07.2019
(51) Int. Cl.: G01R 27/02, A61B 5/0295, A61B 5/113

(54) **DETECTING DEVICE, MEASURING SYSTEM, MONITORING SYSTEM, AND PROGRAM**

(30) Priority: 19.07.2018 WO PCT/JP2018/027196; 16.01.2019 WO PCT/JP2019/001102
(71) Applicant: Posh Wellness Laboratory Inc., Minato-ku, Tokyo 107-0051 (JP)
(72) Inventor: NEBUYA, Satoru, Tokyo 107-0051 (JP)
(74) Representative: Plougmann Vingtoft a/s
(86) International application number: PCT/JP2019/028462
(87) International publication number: WO 2020/017636

(57) **Abstract**

Provided is a detecting device provided with: a substrate; a positive electrode and a negative electrode provided on the substrate; an electric field generating circuit which supplies an electric current or a voltage between the positive electrode and the negative electrode to generate an electric field from a first surface on one side of the substrate; a guard electrode which is provided in a position farther from the first surface than the positive electrode and the negative electrode, and which faces at least one of the positive electrode and the negative electrode with an insulating material interposed therebetween; a potential adjusting circuit which causes the potential applied to the electrode corresponding to the guard electrode, from among the positive electrode and the negative electrode, and the potential of the guard electrode to be the same potential; and a semiconductor element which outputs an electric field intensity detected value corresponding to the electric field intensity between the positive electrode and the negative electrode. Also provided are a measuring system, a monitoring system, and a program.

## Description

### TECHNICAL FIELD

The present disclosure relates to a detection device, a measuring system, a monitoring system, and a program for detecting a state of a living body.

### BACKGROUND ART

Conventionally, there has been known a technique of sending a weak current from an electrode pair attached to a body surface, and imaging a conductivity distribution or a change in the conductivity distribution in a living body from a potential difference generated on the body surface. Patent Document 1 discloses a driver monitoring device capable of monitoring a state of a driver of a vehicle by applying an Electrical Impedance Tomography (hereinafter, EIT) technique to a seat belt.

### PRIOR ART

### PATENT DOCUMENT

Patent Document 1: Japanese Unexamined Patent Application Publication No. 2017-136304

### SUMMARY OF DISCLOSURE

### PROBLEMS TO BE SOLVED BY THE DISCLOSURE

In the prior art, each electrode is connected to a measurement circuit provided near the end of the seat belt, and the measurement circuit relays electrical signal transmitted and received between the driver monitoring device and the electrode. As described above, when the driver wears the seat belt, the electrode pair provided on the seat belt contacts a driver's body surface and the state of the driver can be measured. Thus, in order to detect the state of the living body, the living body has to wear the seat belt or the like.

The present disclosure focuses on these points, and an object of the present disclosure is to conveniently detect a state of a living body.

### MEANS FOR SOLVING THE PROBLEMS

The first aspect of the present disclosure provides a detection device that includes a substrate, wherein the substrate is provided with a positive electrode and a negative electrode, an electric field generating circuit that supplies a current or voltage between the positive electrode and the negative electrode to generate an electric field from a first side of the substrate, a guard electrode that is located farther from the first surface than the positive electrode and the negative electrode, and that faces at least one of the positive electrode and the negative electrode via an insulator, a potential adjustment circuit that makes i) the potential applied to the electrode corresponding to the guard electrode among the positive electrode and the negative electrode and ii) the potential of the guard electrode the same, and a semiconductor element that outputs an electric field intensity detection value corresponding to the electric field intensity between the positive electrode and the negative electrode.

The guard electrode may include a positive guard electrode facing the positive electrode and a negative guard electrode facing the negative electrode.

The substrate includes a first electrode layer provided with the positive electrode and the negative electrode, a second electrode layer provided with the guard electrode, a third electrode layer provided with a reference electrode providing a reference potential, and the first electrode layer, the second electrode layer, and the third electrode layer may be arranged in the order of the first electrode layer, the second electrode layer, and the third electrode layer from the first surface of the substrate to a surface opposite to the first surface.

The substrate may further include a mounting part where at least one of an accelerometer, a gyroscope, and a six-axis sensor is mounted at a position farther from the first surface than the third electrode layer.

The detection device may further include a power supply layer that supplies power to the electric field generating circuit, the potential adjustment circuit, and the semiconductor element. The detection device may further include a first communication part that generates a signal based on the detected electric field intensity value outputted from the semiconductor element and supplies the signal to the outside of the substrate.

The second aspect of the present disclosure provides a measuring system that includes the detection device according to the first aspect that is arranged in a manner that the electric field generated from the positive electrode and the negative electrode reaches at least a portion of a living body, and a measuring device that measures a state of the living body on the basis of the detection result of the detection device.

The measuring device may include a second communication part that communicates with the detection device to receive the signal based on the electric field intensity detection value, and an estimation part that estimates at least one of the movement, heartbeat, and blood pressure of the living body on the basis of the received signal.

The measuring device may further include a prediction part that predicts a future state of the living body on the basis of an estimation result of the estimation part.

The substrate is further provided with a temperature sensor, the detection device transmits a signal based on a detection result of the temperature sensor and the electric field intensity detection value, and the prediction part may predict at least one of a variation in the body temperature of the living body and a balance state of salt and water inside the living body, on the basis of the successive detection results of the temperature sensor.

The measuring system may further includes a seat belt mounted on a seat, wherein the detection device is mounted on the seat belt, and the measuring device may communicate with one or more of the detection devices to measure the state of the living body seated in the seat and fastening the seat belt.

One or more of the detection devices is attached to clothing worn by the living body, and the measuring device may communicate with one or more of the detection devices to measure the state of the living body.

The third aspect of the present disclosure provides a monitoring system that includes the measuring system according to the second aspect, acquisition parts that are connected to the measuring devices and acquire measurement results indicating the state of the living body, and a detection part that detects an abnormality of the living body.

The fourth aspect of the present disclosure provides a program that, when executed by a computer, causes the computer to function as at least a part of the monitoring system according to the third aspect.

### EFFECT OF THE DISCLOSURE

According to the present disclosure, there is an effect of conveniently detecting a state of a living body.

### BRIEF DESCRIPTION OF THE DRAWINGS

FIG. 1 is a drawing for explaining an outline of a monitoring system S according to the present embodiment.
FIG. 2 shows a configuration example of a detection device 10 according to the present embodiment.
FIG. 3 shows a configuration example of a semiconductor element 310 according to the present embodiment.
FIG. 4 shows an example of an intensity distribution of an electric field generated around a positive electrode 210 and a negative electrode 220 according to the present embodiment.
FIG. 5 shows a configuration example of a measuring device 20 according to the present embodiment.
FIG. 6 is a configuration example of the positive electrode 210 and the negative electrode 220 according to the present embodiment.
FIG. 7 is an example of a current density distribution generated around the positive electrode 210 and the negative electrode 220 according to the present embodiment.
FIG. 8 shows how the impedance between the positive electrode 210 and the negative electrode 220 changes with the positive electrode 210 and the negative electrode 220 shown in FIG. 6 mounted on a person's chest.
FIG. 9 shows a configuration of a first variation of an electrode pair E according to the present embodiment.
FIG. 10 shows a configuration of a second variation of the electrode pair E according to the present embodiment.
FIG. 11 shows a configuration example of a guard electrode 230 and a potential adjustment circuit 318 according to the present embodiment.
FIG. 12 shows a variation of the monitoring system S according to the present embodiment.

### DETAILED DESCRIPTION OF THE DISCLOSURE

### [An outline of a monitoring system S]

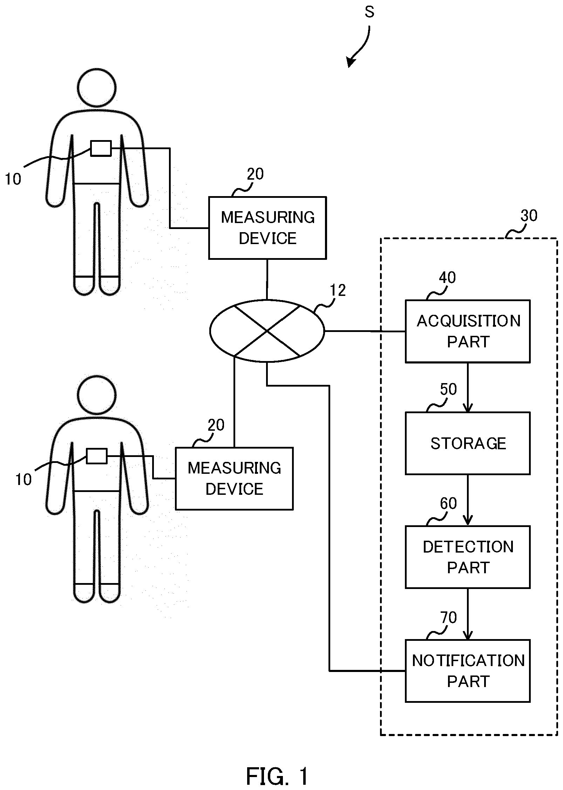

FIG. 1 is a drawing for explaining an outline of a monitoring system S according to the present embodiment. The monitoring system S monitors movement and the like of a living body on the basis of a detection signal transmitted by a detection device 10 carried by the living body. In this embodiment, an example in which the living body is a human body will be described. The monitoring system S includes the detection device 10, a measuring device 20, and a monitoring device 30.

The detection device 10 is carried by a user, and is provided with a sensor for outputting the detection signal that varies according to the state of the user. For example, the user may put the detection device 10 in a pocket or the like of a shirt, jacket, underwear, pajama, hospital gown, lounge wear, or other clothing worn by the user. Further, the detection device 10 may be attached to or mounted on the user's clothing to be carried by the user. The detection device 10 may be attached or mounted on a body surface of the user. The details of the detection device 10 will be described later.

The measuring device 20 measures the state of the user on the basis of a detection result of the sensor included by the detection device 10. The measuring device 20 measures, for example, a state of the movement, breathing, and heartbeat of the user. The measuring device 20 may be attached to the user's clothing or the like, or may alternatively be provided separately from the clothing. The detection device 10 and the measuring device 20 are connected to each other by wire or wirelessly, for example. In the monitoring system S, for example, such a detecting device 10 and a measuring device 20 are provided for each user. Alternatively, the measuring device 20 may be provided for one or more users.

In the monitoring system S, for example, a set of such a detecting device 10 and a measuring device 20 is provided for each user. Specifically, in the monitoring system S, a measurement result based on the detection system 10 and the measuring device 20 is stored in association with information for identifying the user using the detection system 10 and the measuring device 20. The measuring device 20 transmits the measurement result to the monitoring device 30 via, for example, a network 12. FIG. 1 illustrates a plurality of sets of a measuring device 20 and a detection device 10 communicating with the monitoring device 30 via the network 12. The detection device 10 and the measuring device 20 may function as a measuring system for measuring the state of the user.

Alternatively, the detection device 10 may transmit the measurement result to the monitoring device 30 via, for example, the network 12. In this instance, the monitoring device 30 also functions as the measuring device 20. It should be noted that the network 12 may be the Internet, or may be a local area network, instead.

The monitoring device 30 is, for example, a computer such as a server. The monitoring device 30 includes an acquisition part 40, a storage 50, a detection part 60, and a notification part 70. The acquisition part 40 is connected to each of one or more measuring devices 20, and acquires each of the measurement results indicating the state of the user corresponding to the measuring device 20. The acquisition part 40 may be connected to a plurality of measuring devices 20 via the network 12, or may be directly connected to the plurality of measuring devices 20.

The storage 50 stores the acquired measurement results of the plurality of measuring devices 20. Further, the storage 50 may store intermediate data, a calculation result, a threshold, a parameter, and the like, which are generated (or used) by the monitoring system S in the course of the operation of the monitoring system S. The storage 50 may supply the stored data to a requester in response to a request from each part in the monitoring system S.

The detection part 60 detects an abnormal state among the states of one or more users. For example, the detection part 60 detects, among the plurality of users, a user whose state of movement, breathing, heartbeat, and the like is abnormal. In this case, the detection part 60 may detect the duration or the like of the state in which the user is abnormal. For example, the detection part 60 detects the user whose heart rate is less than a threshold value among the plurality of users. In this case, the detection part 60 may detect the duration and the like of the state in which the heart rate of said user is less than the threshold value.

In response to the detection part 60 detecting the user in the abnormal state, the notification part 70 notifies the outside that an abnormal user has been detected. The notification part 70, for example, indicates to the operator of the monitoring system S and the like that an abnormality has been detected, on a display and the like. The notification part 70 may generate a voice and the like to provide notification that an abnormality has been detected. Further, the notification part 70 may notify an external server or the like that an abnormality has been detected, via the network 12. The notification part 70 may also notify the user himself/herself that an abnormality has been detected. In this case, the notification part 70 notifies, for example, a mobile terminal and the like owned by the user.

As described above, since the monitoring system S according to the present embodiment measures the state of the user on the basis of the detection result of the detection device 10 carried by the user, the monitoring system S does not require a large-scale measuring device and the like. Further, since the monitoring system S can monitor the plurality of users using the network 12 and the like, it is possible to measure the state of the user regardless of the location of the user. The monitoring system S can monitor the states of the plurality of users including, for example, a user under home care. The detection device 10 used by the monitoring system S will be described below.

### [A configuration example of the detection device 10]

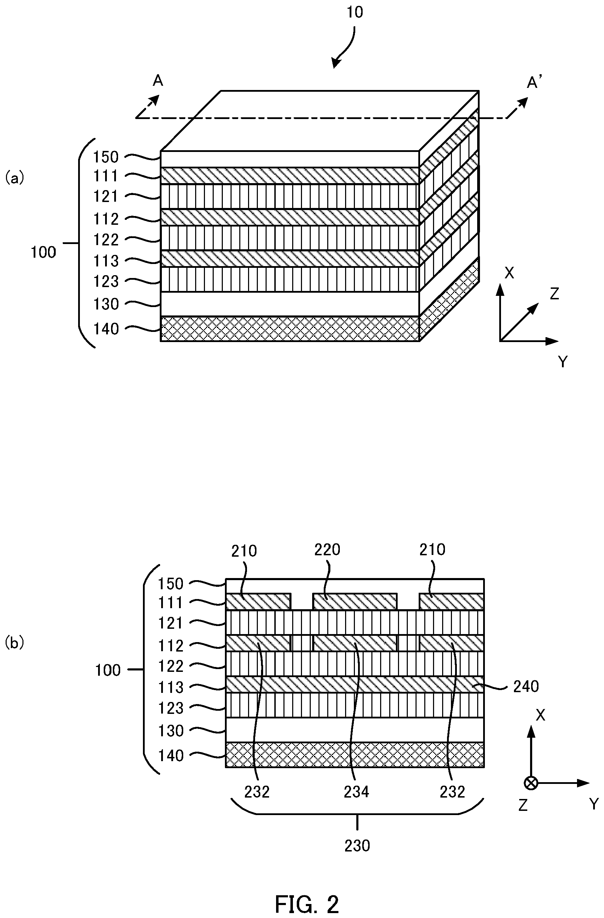

FIG. 2 shows a configuration example of the detection device 10 according to the present embodiment. FIG. 2(a) shows an exemplary perspective view of the detection device 10. FIG. 2(b) shows a configuration example of a cross section of the detection device 10 according to the present embodiment. FIG. 2(b) is an example of an A-A'-line cross section of FIG. 2(a).

The detection device 10 functions as a sensor for detecting the state of the user. The detection device 10 is a sheet-like device that detects an intensity of an electric field near the user's body. The detection device 10 includes a substrate 100. The substrate 100 is, for example, a multi-layer substrate on which an electronic component and the like can be mounted. FIG. 2 illustrates an example in which the substrate 100 is a printed circuit board. Further, the substrate 100 may have a flexible base. In this case, the substrate 100 may be a flexible substrate or a cloth-like member made of fibers. The substrate 100 includes a first electrode layer 111, a second electrode layer 112, a third electrode layer 113, a first insulating layer 121, a second insulating layer 122, a third insulating layer 123, a component layer 130, a power supply layer 140, and a protective film 150.

The first electrode layer 111, the first insulating layer 121, the second electrode layer 112, the second insulating layer 122, the third electrode layer 113, the third insulating layer 123, the component layer 130, and the power supply layer 140 are arranged in the order of the first electrode layer 111, the first insulating layer 121, the second electrode layer 112, the second insulating layer 122, the third electrode layer 113, the third insulating layer 123, the component layer 130, and the power supply layer 140 from a first surface of the substrate 100 toward a surface opposite to the first surface. It should be noted that, in FIG. 2, a surface facing a +X direction of the substrate 100 is the first surface.

The first electrode layer 111 is provided with a positive electrode 210 and a negative electrode 220. The positive electrode 210 and the negative electrode 220 constitute an electrode pair E that generates an electric field. The positive electrode 210 and the negative electrode 220 are laminated on a surface of the first insulating layer 121 facing the +X direction. FIG. 2 shows an example in which the protective film 150 covers portions of the positive electrode 210, the negative electrode 220, and the first insulating layer 121 in the first surface of the substrate 100. Further, the protective film 150 may be provided so as to cover the entire substrate 100. Alternatively, portions of the positive electrode 210, the negative electrode 220, and the first insulating layer 121 may be exposed.

The electrode pair E constituted by the positive electrode 210 and the negative electrode 220 is an example of an electric element whose electrical characteristics change according to the movement of the user carrying the detection device 10. For example, the impedance in the vicinity of the electrode pair E changes according to the movement based on the state of the user's body such as breathing or a heartbeat. As a result, the state of the electric field generated by the electrode pair E changes, and so the potential difference between the electrodes constituting the electrode pair E changes. The detection device 10 detects such a change in the impedance between the electrodes constituting the electrode pair E to output a detection value indicating a change in the intensity of the electric field in the vicinity of the electrode pair E.

The second electrode layer 112 is provided with a guard electrode 230. The guard electrode 230 is provided at a position farther from the first surface than the positive electrode 210 and the negative electrode 220, and faces at least one of the positive electrode 210 and the negative electrode 220 via an insulator. For example, the guard electrode 230 faces the positive electrode 210 and the negative electrode 220 with the first insulating layer 121 interposed therebetween. The guard electrode 230 is laminated on a surface of the second insulating layer 122 facing the +X direction.

The guard electrode 230 includes, for example, a positive guard electrode 232 facing the positive electrode 210. In this case, the positive guard electrode 232 is formed in substantially the same shape as the positive electrode 210, as an example. A potential substantially the same as the potential applied to the positive electrode 210 is applied to the positive guard electrode 232. Further, the guard electrode 230 includes, for example, a negative guard electrode 234 facing the negative electrode 220. In this case, the negative guard electrode 234 is formed in substantially the same shape as the negative electrode 220, as an example. A potential substantially the same as the potential applied to the negative electrode 220 is applied to the negative guard electrode 234.

The third electrode layer 113 is provided with a reference electrode 240 providing a reference potential. The reference potential is, for example, a ground potential such as 0 V. The reference electrode 240 is laminated on a surface of the third insulating layer 123 facing the +X direction. The reference electrode 240 is formed to cover the surface of the third insulating layer 123 facing the +X direction, as an example.

The component layer 130 contains a circuit for generating the electric field from the electrode pair E, a semiconductor element for detecting a change in the electrical characteristics of the electrode pair E, and the like. The component layer 130 is, for example, a member in which a circuit, an element, and the like are provided in an insulating material of the substrate and the like. The component layer 130 is composed of one or more layers. The circuit and the element provided in the component layer 130 will be described later.

The power supply layer 140 supplies power to the circuit, the element, and the like mounted on the detection device 10. The power supply layer 140 preferably includes a thin-film battery that can be recharged. More preferably, the battery is removable from the substrate 100. FIG. 2 shows the component layer 130 and the power supply layer 140 as separate layers, but they are not limited thereto. The component layer 130 and the power supply layer 140 may be included in one layer. Further, the order in which the component layer 130 and the power supply layer 140 are laminated may be reversed.

As described above, the detection device 10 according to the present embodiment includes the substrate 100 having a multi-layer structure, the positive electrode 210, the negative electrode 220, the guard electrode 230, and the reference electrode 240 provided in the substrate 100, as well as the circuits and elements connected to these electrodes. The detection device 10 outputs, to the measuring device 20, an electric field intensity detection value indicating a value corresponding to the intensity of the electric field between the electrodes constituting the electrode pair E.

The electric field intensity detection value is not limited to the value of the intensity of the electric field itself, and can be any value as long as it is a value that changes according to the electric field intensity, such as an impedance value or a combination of a current value and a resistance value. The detection device 10 may output a signal processed or treated on the basis of the intensity of the electric field to the measuring device 20. The circuit and the element provided in such a detection device 10 will be described below.

### [A configuration example of a semiconductor element 310]

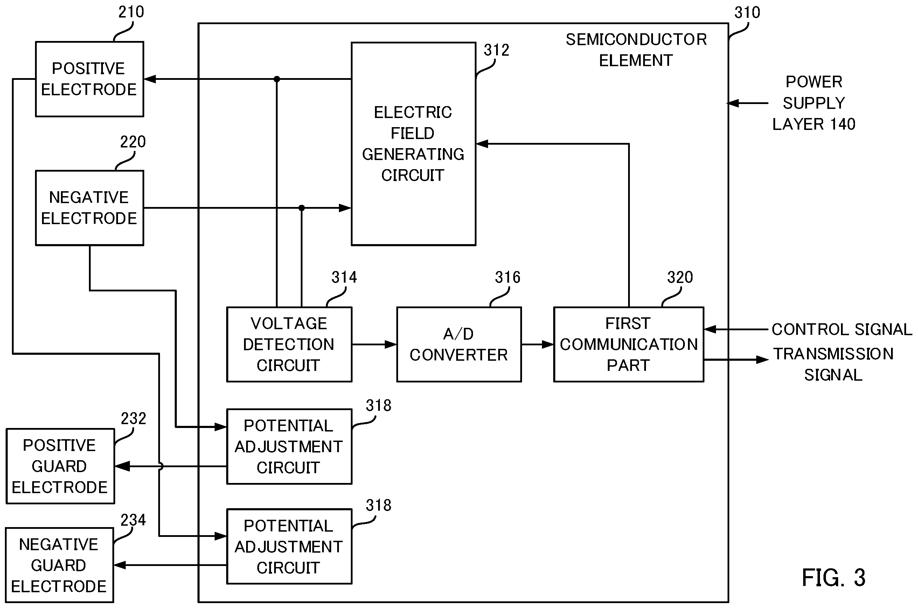

FIG. 3 shows a configuration example of a semiconductor element 310 according to the present embodiment. The semiconductor element 310 is an example of an integrated circuit provided in the component layer 130. The semiconductor element 310 outputs the electric field intensity detection value corresponding to the electric field intensity between the positive electrode 210 and the negative electrode 220. The semiconductor element 310 includes an electric field generating circuit 312, a voltage detection circuit 314, an A/D converter 316, potential adjustment circuits 318, and a first communication part 320.

The electric field generating circuit 312 supplies a current or voltage between the positive electrode 210 and the negative electrode 220 to generate the electric field from a first surface of the substrate 100. The electric field generating circuit 312, for example, supplies a weak current to the positive electrode 210 to generate the electric field between the positive electrode 210 and the negative electrode 220. For example, when a displacement current flows between the positive electrode 210 and the negative electrode 220, a line of magnetic force (magnetic field) corresponding to the displacement current is generated between the positive electrode 210 and the negative electrode 220, and so a line of electric force (electric field) corresponding to the line of magnetic force is further generated. Then, the magnetic field and the electric field are repeatedly generated in the direction away from the first surface of the substrate 100, and the radio wave is propagated. Thus, the electric field generating circuit 312 supplies the current or voltage between the positive electrode 210 and the negative electrode 220 to generate the electric field and the displacement current in the space. The electric field generating circuit 312 determines the timing for supplying the weak current and the current value on the basis of, for example, control data received from the outside.

The voltage detection circuit 314 detects the potential difference between the positive electrode 210 and the negative electrode 220. The voltage detection circuit 314 detects a change in the potential difference of the electrode pair E while, for example, the electric field generating circuit 312 is causing an approximately constant current to flow through the electrode pair E. The potential difference of the electrode pair E has a magnitude corresponding to the change in the impedance in the vicinity of the electrode pair E, and the greater the impedance is, the greater the potential difference becomes, for example. The voltage detection circuit 314 supplies, to the A/D converter 316, a signal of a voltage corresponding to the potential difference between the positive electrode 210 and the negative electrode 220.

The A/D converter 316 converts the analog signal received from the voltage detection circuit 314 into a digital signal. That is, the A/D converter 316 generates digital data corresponding to the voltage value of the signal outputted by the voltage detection circuit 314. The A/D converter 316 supplies the generated digital signal to the first communication part 320.

The potential adjustment circuit 318 makes the potential of the electrode corresponding to the guard electrode 230 among the positive electrode 210 and the negative electrode 220 the same as the potential of the guard electrode 230. The potential adjustment circuit 318 makes, for example, the positive electrode 210 and the positive guard electrode 232 have the same potential while maintaining the insulation. Further, the potential adjustment circuit 318 makes, for example, the negative electrode 220 and the negative guard electrode 234 have the same potential while maintaining the insulation.

The first communication part 320 generates, on the basis of the digital signal received from the A/D converter 316, a transmission signal based on the electric field intensity detection value outputted by the semiconductor element 310, and supplies it to the outside of the substrate 100. The first communication part 320 can communicate with the outside, either by wire or wirelessly. The first communication part 320 communicates with the measuring device 20 via a wireless channel, such as Bluetooth (registered trademark), for example.

Further, the first communication part 320 receives the control signal transmitted from the outside, and supplies said control signal to the inside of the substrate 100. For example, the first communication part 320 receives the control signal by a predetermined communication method as described above from the measuring device 20, and supplies said control signal to the electric field generating circuit 312.

The above-described semiconductor element 310 operates using the power supplied from the power supply layer 140. In this case, the power supply layer 140 supplies the power to, for example, the electric field generating circuit 312, the voltage detection circuit 314, the A/D converter 316, the potential adjustment circuit 318, and the first communication part 320. It should be noted that FIG. 3 shows an example of one semiconductor element 310 provided in the component layer 130, but it is not limited thereto. One or more members among the electric field generating circuit 312, the voltage detection circuit 314, the A/D converter 316, the potential adjustment circuit 318, and the first communication part 320 may be formed as different semiconductor elements and mounted on the component layer 130. The electric field radiated to the user by such a semiconductor element 310 will be described below.

### [An intensity distribution of the electric field generated by the electrode pair E]

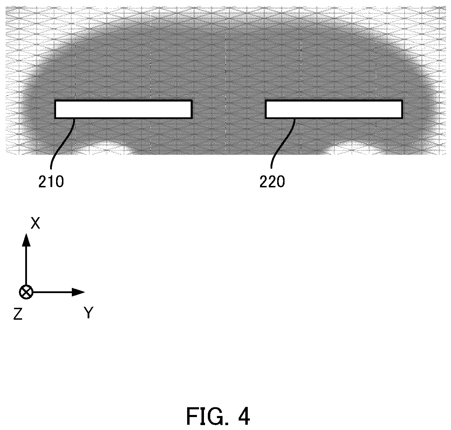

FIG. 4 shows an example of an intensity distribution of the electric field generated around the positive electrode 210 and the negative electrode 220 according to the present embodiment. FIG. 4 shows an example of the electrode pair E in which the positive electrode 210 and the negative electrode 220 are aligned in parallel. In FIG. 4, the electric field intensity in the dark-colored region is greater than the electric field intensity in the light-colored region. It can be seen that the electric field generating circuit 312 supplies the weak current to the positive electrode 210, resulting in a strong electric field region between the positive electrode 210 and the negative electrode 220.

For example, the user's breathing and heartbeat moves the user's chest. In addition, the magnitude of the current flowing through the body changes in synchronization with a) the impedance change of the blood flow due to the heartbeat or b) the impedance change of the lungs due to the breathing. When the electric field generated by the positive electrode 210 and the negative electrode 220 is supplied to the region where such a change occurs, the intensity level of the electric field generated between the positive electrode 210 and the negative electrode 220 changes in accordance with the user's breathing and heartbeat.

When the electric field intensity changes, the potential difference between the positive electrode 210 and the negative electrode 220 changes. The measuring device 20 can determine the state of the user's breathing and the heartbeat on the basis of the change in the potential difference between the positive electrode 210 and the negative electrode 220 detected by the voltage detection circuit 314. In addition, the monitoring device 30 can also monitor the occurrence of abnormalities according to the determined state of the user.

As described above, the first surface of the detection device 10 is arranged to face the user such that the electric field generated from the positive electrode 210 and the negative electrode 220 reaches at least a part of the user's body. Arranging the detection device 10 at a position where the electric field can be generated in a region, such as the user's chest, where the impedance and the like changes in accordance with the state of the user enables the monitoring system S to conveniently monitor the state of the user.

As described in FIG. 2, the detection device 10 is formed as a substrate 100 having a multi-layer structure. Such a layer structure can be formed, for example, in a plate shape having a thickness of about several millimeters or less. Further, the size of the substrate 100 may be, for example, about the size of a card that can be accommodated in a user's breast pocket or the like. Therefore, just by carrying the detection device 10 in the pocket of the user's clothing or the like, the monitoring system S can monitor the state of said user.

In this case, it is desirable that the detection device 10 is arranged so that the user's body is located to the +X direction side of the positive electrode 210 and the negative electrode 220 in FIG. 4. As also shown in FIG. 4, the electric field generated between the positive electrode 210 and the negative electrode 220 is also generated toward a -X direction. Such an electric field directed in the -X direction cannot be used as the electric field for detecting the state of the user. Further, the electric field directed in the -X direction may affect, as noise, the circuit, the elements, and the like provided on the +X direction side of the positive electrode 210 and the negative electrode 220.

Therefore, the detection device 10 is provided with the guard electrode 230 corresponding to the positive electrode 210 and/or the negative electrode 220 to reduce such an electric field directed in the -X direction. Further, the detection device 10 is further provided with the third electrode layer 113 providing a reference potential on the -X direction side of the guard electrode 230. Thus, in the component layer 130 provided on the -X direction side of the third electrode layer 113, the electric field generated by the electrode pair E can be reduced.

An example in which the above-described detection device 10 is provided with one electrode pair E constituted by the positive electrode 210 and the negative electrode 220 has been described, but it is not limited thereto. The detection device 10 may be provided with a plurality of electrode pairs E. In this case, the component layer 130 may be provided with a plurality of semiconductor elements 310 corresponding to the plurality of electrode pairs E.

When the plurality of semiconductor elements 310 is formed in the component layer 130, each of the semiconductor elements 310 is preferably provided at a position closer to its corresponding electrode pair E than the electrode pair E corresponding to the other semiconductor elements 310. Further, a plurality of distances between each of the plurality of semiconductor elements 310 and the electrode pair E corresponding respectively to the plurality of semiconductor elements 310 are desirably the same. That is, in a plurality of sets of a semiconductor element 310 and an electrode pair E, the distance between the semiconductor element 310 and its corresponding electrode pair E (a set of the positive electrode 210 and the negative electrode 220) is constant. Said distance is a distance between, for example, a) the center position of the semiconductor element 310 and b) an intermediate position between the center position of the positive electrode 210 included in the electrode pair E and the center position of the negative electrode 220 included in the electrode pair E.

Since the distances between the plurality of semiconductor elements 310 and their respective corresponding electrode pairs E are constant as described above, the detection sensitivity of the electric field intensity of each of the plurality of semiconductor elements 310 (that is, the magnitude of the electric field intensity detection value of the electric field intensity) is approximately the same. Therefore, the detection device 10 can detect the change in the electric field with high accuracy regardless of its position in the substrate 100.

Further, each of the plurality of semiconductor elements 310 transmits, for example, the digital data indicating the electric field intensity detection value from the shared first communication part 320 to the measuring device 20. Each of the plurality of semiconductor elements 310 transmits the digital data including the electric field intensity detection value to the measuring device 20 at the time when, for example, each of the plurality of semiconductor elements 310 receives a command with an address of the semiconductor elements 310 from the measuring device 20.

The plurality of semiconductor elements 310 measures the potential differences between the positive electrodes 210 and the negative electrodes 220 simultaneously on the basis of, for example, the control signal inputted from the measuring device 20. Each of the plurality of semiconductor elements 310 temporarily holds the electric field intensity detection value indicating the measured electric potential difference until when the semiconductor element 310 itself transmits the electric field intensity detection value, and then transmits the digital data including the electric field intensity detection value to the measuring device 20 at the timing when the semiconductor element 310 itself transmits the electric field intensity detection value.

Alternatively, or in addition, the user may carry a plurality of detection devices 10 at different positions on the body. In this way, the measuring device 20 can identify, at each measurement timing, the state of the user's body in the vicinity of different positions at the same time. Such a measuring device 20 will be described below.

### [A configuration of the measuring device 20]

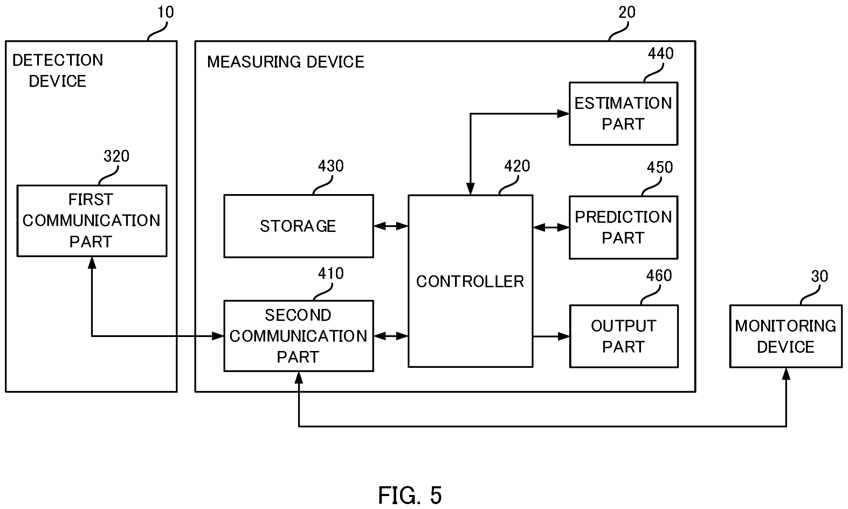

FIG. 5 shows a configuration example of the measuring device 20 according to the present embodiment. The measuring device 20 measures the state of the living body such as a user on the basis of the signal transmitted from the first communication part 320 in the detection device 10. The measuring device 20 includes a second communication part 410, a controller 420, a storage 430, an estimation part 440, a prediction part 450, and an output part 460.

The second communication part 410 transmits and receives a signal to and from the detection device 10. The second communication part 410 receives, for example, the signal based on the electric field intensity detection value transmitted by the first communication part 320. The second communication part 410 notifies the controller 420 of, for example, the digital data received from one or more first communication parts 320. Here, the second communication part 410 may notify the controller 420 of the digital data received from the first communication part 320 in association with identification information for identifying the semiconductor element 310. The second communication part 410 also transmits a signal for controlling the detection device 10 to the first communication part 320.

The second communication part 410 may further transmit and receive a signal to and from the monitoring device 30. The second communication part 410 transmits, for example, the measurement result of the measuring device 20 to the monitoring device 30. Further, the second communication part 410 may also receive a signal for controlling the detection device 10 from the monitoring device 30.

The second communication part 410 communicates with each of the detection device 10 and the monitoring device 30 by wire and/or wirelessly. The second communication part 410 transmits and receives data by, for example, a wireless LAN such as Wi-Fi (registered trademark), Bluetooth (registered trademark), mobile communication, and the like. The second communication part 410 may transmit and receive data to and from external databases or the like.

The controller 420 stores the digital data received by the second communication part 410 in the storage 430. The controller 420 controls the operation timing and the like in the measuring device 20. For example, the controller 420 controls the communication timing of the second communication part 410. Furthermore, the controller 420 controls the measuring timing of the measuring device 20, the transmission timing of the measurement result, and the like. The controller 420 includes a CPU, for example.

The storage 430 stores the digital data received by the second communication part 410. Moreover, the storage 430 may store intermediate data, a calculation result, a threshold, a parameter, and the like, which are generated (or used) by the measuring device 20 in the course of operation. In addition, the storage 430 may provide the stored data to the requester in response to a request from each unit in the measuring device 20. The storage 430 includes, for example, a ROM and a RAM, and also a program if the controller 420 executes the program and the like.

The estimation part 440 estimates at least one of the user's movement, heartbeat, and blood pressure on the basis of the received signal received by the second communication part 410. The estimation part 440 calculates, for example, the temporal variation of the impedance on the basis of the change in the electric field intensity, and estimates the state of the user in accordance with the temporal variation of the impedance. In this instance, the estimation part 440 estimates the state of the user according to an impedance variation cycle and/or an impedance variation range.

Further, the estimation part 440 may estimate the state of the user by comparing a) the relationship between the previously-measured state of the user and the change of the impedance with b) the temporal variation of the impedance. In this instance, the storage 430 preferably stores in advance the relationship between the state of the user and the change of the impedance. The estimation part 440 estimates the state of the user, such as breathing-induced body and/or lung variation, heartbeat-induced body variation, heart rate-induced blood flow variation, and the like, corresponding to the change of the impedance. The estimation part 440 estimates the state of the user on the basis of, for example, an instruction from the controller 420.

The prediction part 450 predicts the future state of the user on the basis of the estimation result of the estimation part 440. The prediction part 450 predicts whether the user is sleeping, is transitioning to sleep, or is transitioning from sleep to awakening on the basis of, for example, an estimation result of the breathing, heartbeat, and/or blood flow of the user. In addition, the prediction part 450 may predict whether the user is in a sleep apnea state, is transitioning to an apnea state, or is transitioning from an apnea state to a respiratory state.

Further, the prediction part 450 may predict the user's health condition, future health condition, and the like on the basis of the results of the periodic estimation of the state of the user by the estimation part 440. The prediction part 450 may predict the state of the user on the basis of statistical information stored outside or inside. The prediction part 450 predicts the state of the user on the basis of, for example, an instruction from the controller 420.

The output part 460 outputs the estimation result of the estimation part 440 and/or the prediction result of the prediction part 450. The output part 460 may cause a display device, such as a display, to display the estimation result and the prediction result. The output part 460 may store the estimation result and the prediction result in the storage 430. The output part 460 outputs the estimation result and the prediction result on the basis of, for example, an instruction from the controller 420.

The measuring device 20 according to the present embodiment is desirably formed of an integrated circuit and the like. Further, the measuring device 20 is more preferably to be configured as a mobile-type portable device equipped with a battery or the like. This allows the measuring device 20 to be easily held in the user's pocket, belt, bag, and the like. In this instance, the user can carry the detection device 10 and the measuring device 20.

Furthermore, the measuring device 20 may be configured as a part of a terminal carried by the user. The terminal is, for example, a communicable device such as a mobile phone, a smart phone, a game machine, a tablet PC, a small PC, a notebook PC, and the like. It should be noted that, when the measuring device 20 is a portable device such as a mobile phone with at least one surface facing the user's body, the detection device 10 and the measuring device 20 may be formed as an integrated device.

Moreover, the measuring device 20 may function as the monitoring device 30 if the measuring device 20 has a processing capability sufficient to execute data processing of the measurement result. In this instance, the measuring device 20 and the monitoring device 30 are formed as an integrated device. In addition, the detection device 10, the measuring device 20, and the monitoring device 30 may be formed as an integrated device.

Since the guard electrode 230 of the detection device 10 has substantially the same potential as that of the corresponding electrode of the electrode pair E, the electric field leaking from the surface of the substrate 100 opposite to the first surface is reduced among the electric fields generated by the electrode pair E. Thus, the detection device 10 can reduce the occurrence of the noise and the like while efficiently outputting the electric field from the first surface to the user and reducing the leakage of the electric field to the surface opposite to the first surface. Therefore, the detection device 10 can prevent the occurrence of the noise affecting the measuring device 20 even when the detection device 10 is, for example, integrated with the measuring device 20.

Since the detection device 10 can be formed with a small size as described above, the monitoring system S according to the present embodiment can conveniently detect the state of the user when the user carries the detection device 10 with its first surface facing his/her body. The positive electrode 210 and the negative electrode 220 provided in the detection device 10 will be described below.

### [The positive electrode 210 and the negative electrode 220]

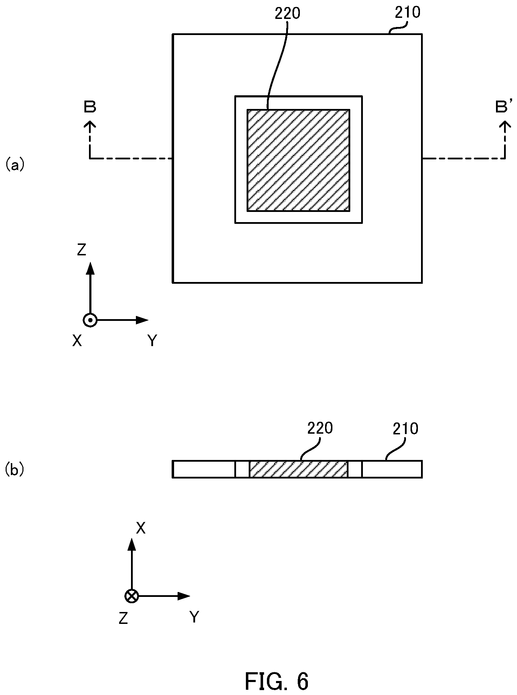

FIG. 6 is a configuration example of the positive electrode 210 and the negative electrode 220 according to the present embodiment. FIG. 6(a) is a plane figure of the positive electrode 210 and the negative electrode 220, and FIG. 6(b) is a B-B'-line cross section. The positive electrode 210 shown in FIG. 6 is square in profile and has a square void area capable of accommodating the negative electrode 220 therein, but the contour shape of the positive electrode 210 and the shape of the inner void area are arbitrary.

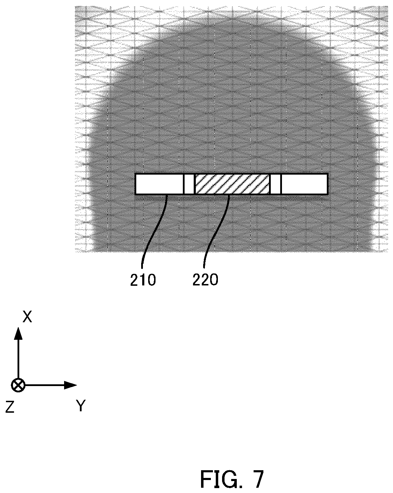

FIG. 7 is an example of a current density distribution generated around the positive electrode 210 and the negative electrode 220 according to the present embodiment. The current density distribution is proportional to the intensity distribution of the electric field occurring around the positive electrode 210 and the negative electrode 220. In FIG. 7, the current density in the dark-colored region is greater than the current density in the light-colored region. It can be seen that the electric field generator 131 supplies the weak current to the positive electrode 210, resulting in a strong electric field region between the positive electrode 210 and the negative electrode 220. It should be noted that the positional relationship between the positive electrode 210 and the negative electrode 220 may be reversed, that is, the negative electrode 220 may surround the positive electrode 210.

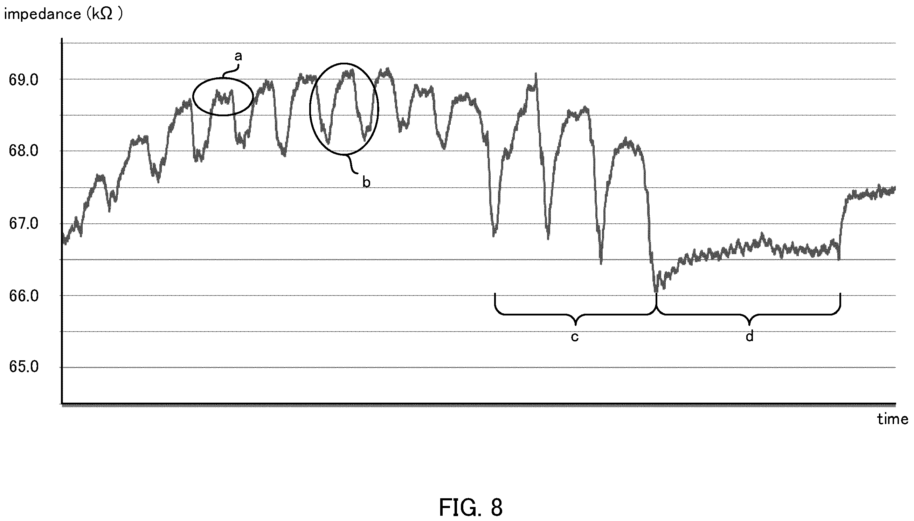

FIG. 8 shows how the impedance between the positive electrode 210 and the negative electrode 220 changes with the positive electrode 210 and the negative electrode 220 shown in FIG. 6 mounted on a person's chest. FIG. 8 illustrates an example of measuring the potential difference between the positive electrode 210 and the negative electrode 220 while a weak current having a frequency of 1 MHz was flowing through the positive electrode 210 with the positive electrode 210 and the negative electrode 220 attached to clothes on the person's chest. Then, the impedance was calculated on the basis of i) the magnitude of the weak current (current value) flowing through the positive electrode 210 and the negative electrode 220 and ii) the measured potential difference. The contour shape of the positive electrode 210 used was a square having a side of 40 mm.

FIG. 8 shows the change in the impedance in synchronization with the heartbeat (a in FIG. 8) and the change in the impedance in synchronization with the breathing (b in FIG. 8). It can also be confirmed that the amount of the change in the impedance increases when the person takes a deep breath (c in FIG. 8), and the amount of the change in the impedance decreases while the person holds his/her breath (d in FIG. 8). An example in which the electrode pair E according to the present embodiment described above is formed in the shape shown in FIG. 6 has been described, but the electrode pair E is not limited thereto. The electrode pair E may be formed in any shape as long as the electric field can be applied to the user's body.

### [First variation of the electrode pair E]

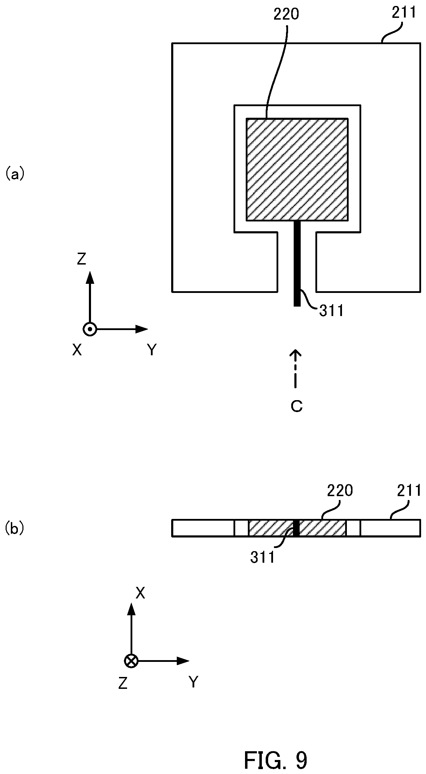

FIG. 9 shows a configuration of a first variation of the electrode pair E according to the present embodiment. FIG. 9(a) is a plane figure of a positive electrode 211 and the negative electrode 220 according to the first variation, and FIG. 9(b) is the positive electrode 211 and the negative electrode 220 viewed from the direction C. The positive electrode 211 surrounds the negative electrode 220 in a region excluding a position where wiring 311 for connecting the negative electrode 220 and the semiconductor element 310 is provided. The positive electrode 211 is not provided at a position where the wiring 311 is provided, and so the wiring 311 does not overlap the positive electrode 211. If the wiring 311 and the positive electrode 211 overlap, the electric field occurs in a region where the wiring 311 and the positive electrode 211 overlap, which leads to the loss of energy. Since the negative electrode 220 and the positive electrode 211 are configured as shown in FIG. 9, the detection device 10 can suppress the loss of energy, and so the measurement accuracy of the potential difference between the positive electrode 211 and the negative electrode 220 can be improved.

### [Second variation of the electrode pair E]

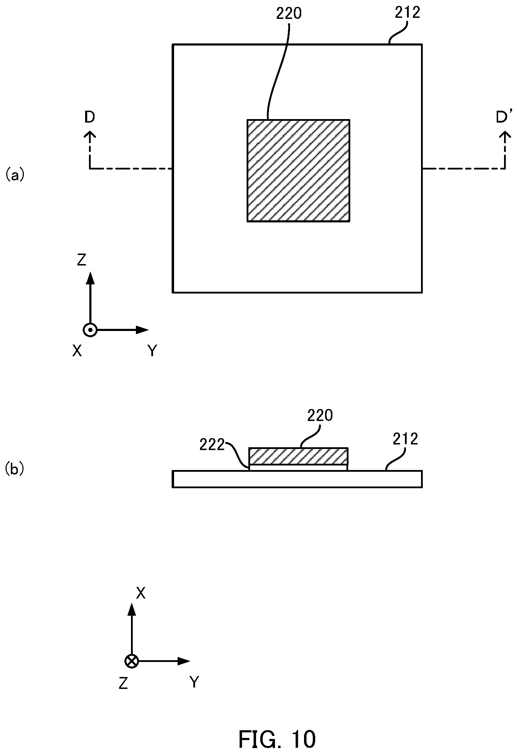

FIG. 10 shows a configuration of a second variation of the electrode pair E according to the present embodiment. FIG. 10(a) is a plane figure of a positive electrode 212 and the negative electrode 220 according to the present variation, and FIG. 10(b) is a D-D'-line cross section thereof. The positive electrode 212 has a square shape, and the negative electrode 220 is provided above the positive electrode 212 with an insulating member 222 interposed therebetween. Even with such a configuration, an electric field equivalent to that of the positive electrode 210 and the negative electrode 220 shown in FIG. 6 can be generated.

### [The guard electrode 230]

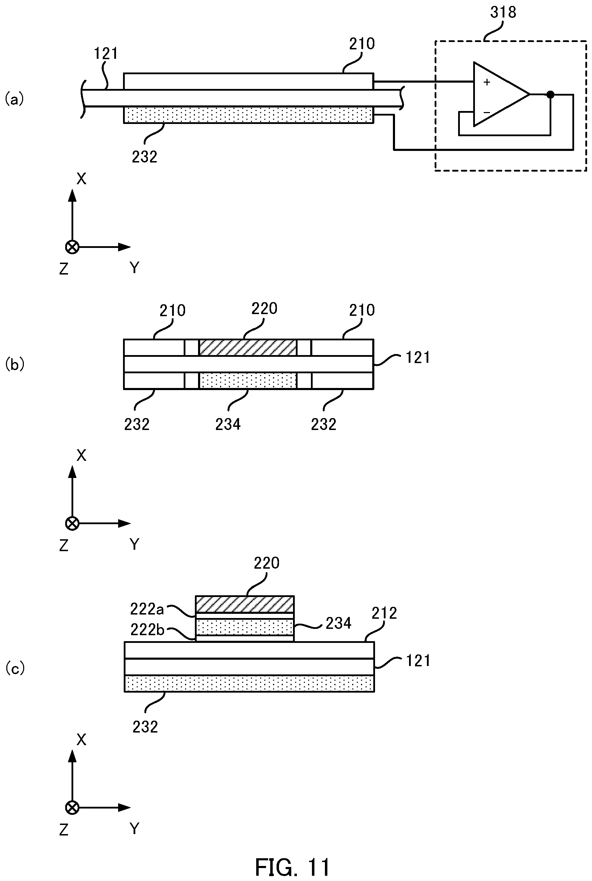

FIG. 11 shows a configuration example of the guard electrode 230 and the potential adjustment circuit 318 according to the present embodiment. As an example, FIG. 11(a) shows an example in which the positive guard electrode 232 is provided on the surface of the first insulating layer 121 opposite to the positive electrode 210 formed on the first surface. The positive guard electrode 232 is connected to the potential adjustment circuit 318 having an operational amplifier.

The potential adjustment circuit 318 in the embodiment shown in FIG. 11(a) is a voltage follower. The positive electrode 210 is connected to a positive input terminal of the operational amplifier, and the positive guard electrode 232 is connected to an output terminal of the operational amplifier, and so the potential of the positive guard electrode 232 is equal to the potential of the positive electrode 210. Although not shown in FIG. 11(a), the potential adjustment circuit 318 may further include a circuit, having a similar circuit configuration, for equalizing the potential of the negative electrode 220 and the potential of the negative guard electrode 234.

FIG. 11(b) shows an example in which the positive guard electrode 232 and the negative guard electrode 234 respectively face the positive electrode 210 and the negative electrode 220 shown in FIG. 6. FIG. 11(c) shows an example in which the positive guard electrode 232 and the negative guard electrode 234 respectively face the positive electrode 212 and the negative electrode 220 shown in FIG. 10. In the embodiment shown in FIG. 11(c), the negative guard electrode 234 is provided between the positive electrode 212 and the negative electrode 220, with an insulator 222a and an insulator 222b interposed therebetween.

Although not shown in FIGS. 11(b) and 11(c), the detection device 10 further includes a) a potential adjustment circuit 318 for making the potential of the positive guard electrode 232 equal to the potential of the positive electrode 210, and b) a potential adjustment circuit 318 for making the potential of the negative guard electrode 234 equal to the potential of the negative electrode 220. Since the guard electrode 230 and the potential adjustment circuit 318 are provided in the detection device 10 in this manner, the electric field generated by the current flowing through the positive electrode 210 and the negative electrode 220 does not leak to the opposite surface of the substrate 100, so that the electric field intensity on the first surface of the substrate 100 on which the positive electrode 210 and the negative electrode 220 are provided is increased. As a result, the monitoring system S can improve the accuracy in detecting the displacement amount of the living body.

It should be noted that the shape of the electrode is not limited to a square or a rectangle, and may be a circle, an ellipse, or a polygon other than a square or a rectangle. Further, the detection device 10 may further include a shield layer (ground potential layer) on a rear surface of at least one of the positive electrode 210, the negative electrode 220, and the guard electrode 230.

As described above, the detection device 10 according to the present embodiment includes the guard electrode 230, and so the noise that affects the circuit and the element provided in the component layer 130 can be reduced. Therefore, even when other types of sensors and the like are mounted on the component layer 130, it is possible to operate the sensors without reducing the detection accuracy of the sensors due to the noise and the like.

Therefore, the substrate 100 may be further provided with a temperature sensor as an example of other types of sensors. In this case, the first communication part 320 transmits a signal based on the detection result of the temperature sensor and the electric field intensity detection value to the measuring device 20. Further, the substrate 100 may be provided with a plurality of temperature sensors. For example, the plurality of temperature sensors are individually placed in the user's trunk direction and external direction through insulating materials to detect the difference between the external air temperature and the body temperature.

These allow the prediction part 450 to further predict the variation of the user's body temperature on the basis of the successive detection results of the temperature sensor. In addition, the prediction part 450 may predict the balance state of salt and water in the body on the basis of the variation of the user's body temperature and other factors over time. This allows the monitoring system S to prevent, for example, the user from suffering from heat stroke and the like.

As described above, the detection device 10 can be equipped with other types of sensors to transmit various detection results to the measuring device 20. For example, the substrate 100 may be further provided with a mounting part in which at least one of an accelerometer, a gyroscope, a six-axis sensor, and the like is mounted at a position farther from the first surface than the third electrode layer 113. It should be noted that, in addition to or instead of such sensors for detecting relative momentum and positional variation, the mounting part may be equipped with a sensor for detecting absolute positional information, such as a GPS sensor, a Wi-Fi module, or the like. Further, the mounting part may also be equipped with a magnetic sensor or the like that detects the change in intensity of the displacement current generated in the detection space as the intensity of the magnetic field. The mounting part may be provided on the component layer 130, or alternatively, it may be provided on the surface of the substrate 100 opposite to the first surface.

Further, the magnetic sensor may be used instead of the voltage detection circuit 314. In this case, the semiconductor element 310 outputs a magnetic field intensity detection value generated by detecting a change in the magnetic field intensity corresponding to the change in the electric field intensity between the positive electrode 210 and the negative electrode 220.

As described above, when the displacement current flows between the positive electrode 210 and the negative electrode 220, the magnetic field corresponding to the displacement current is generated between the positive electrode 210 and the negative electrode 220, and the electric field corresponding to the generated magnetic field is further generated. Then, the magnetic field and the electric field are repeatedly generated in the direction away from the first surface of the substrate 100, and the radio wave is propagated.

If there is a material with impedance in the space where the radio wave is propagated, the electric field intensity changes according to the magnitude of said impedance. Then, the magnetic field also changes according to the change in the electric field. The magnetic sensor detects such a change in the magnetic field intensity corresponding to the change in the electric field intensity. The magnetic sensor is a highly sensitive magnetic sensor that can detect a weak magnetic field in units such as µT (microtesla), nT (nanotesla), pT (picotesla), and fT (femtotesla), for example.

The detection of the potential difference between the positive electrode 210 and the negative electrode 220 by the voltage detection circuit 314 has a sensitivity of about 1/r² when the distance to the position to be measured is r. In contrast, when such a magnetic sensor is used to directly measure the change in the magnetic field, the sensitivity is about 1/r, so the change in the impedance can be detected efficiently.

As described above, the detection device 10 can be formed with a small size. Therefore, one or more of the detection devices 10 may be attached to the clothing worn by the user. The measuring device 20 then communicates with the one or more detection devices 10 to measure the state of the user. In this case, the detection device 10 should be formed in a card shape.

Here, the detection device 10 may be integrated with other types of cards. For example, the detection device 10 can be integrated with a card that is used for a purpose different from that of detecting the state of the user, such as an ID card, prepaid card, credit card, point card, commuter pass, patient's registration card, and the like. This makes it easy for the user to carry the detection device 10 and perform continuous measurements over a long period of time, for example.

The monitoring system S described above allows the detection device 10 to be carried by the user, so that, for example, the state of the user can be detected even when the user is at home or when the user goes out and travels by train, car, and the like. Alternatively, or in addition to this, the detection device 10 may be mounted on a jig or another device that is worn by the user.

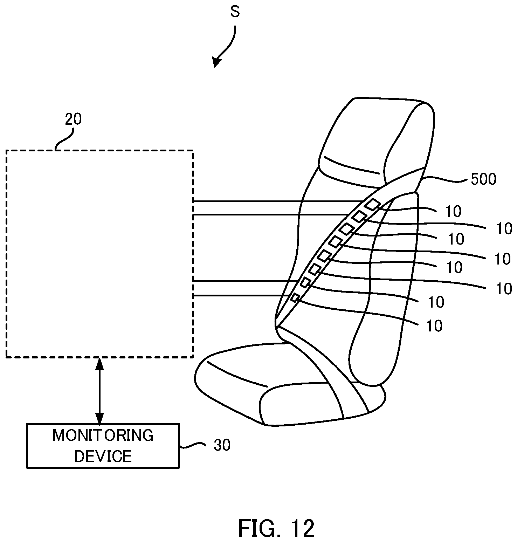

FIG. 12 shows a variation of the monitoring system S according to the present embodiment. In the monitoring system S of the variation, the one or more detection devices 10 are mounted on a seat belt 500 mounted on a seat. The measuring device 20 communicates with the one or more detection devices 10 to measure the state of the user who is seated in the seat and fastening the seat belt. In this case, for example, since the plurality of detection devices 10 can be easily brought into close contact with the user's body, the changes in the impedance at a plurality of positions can be accurately detected, and the measurement accuracy of the measuring device 20 can be improved.

The measuring device 20 is provided inside the vehicle, for example, where the seat belt 500 is provided. Further, the monitoring device 30 is, for example, provided outside the vehicle. Such a monitoring system S can easily monitor the state of the user remotely while the user is traveling in the vehicle.

The present disclosure is explained on the basis of the exemplary embodiments. The technical scope of the present disclosure is not limited to the scope explained in the above embodiments and it is possible to make various changes and modifications within the scope of the disclosure. For example, the specific embodiments of the distribution and integration of the apparatus are not limited to the above embodiments, all or part thereof, can be configured with any unit which is functionally or physically dispersed or integrated. Further, new exemplary embodiments generated by arbitrary combinations of them are included in the exemplary embodiments of the present disclosure. Further, effects of the new exemplary embodiments brought by the combinations also have the effects of the original exemplary embodiments.

### [Description of the reference numerals]

- 10: detection device
- 12: network
- 20: measuring device
- 30: monitoring device
- 40: acquisition part
- 50: storage
- 60: detection part
- 70: notification part
- 100: substrate
- 111: first electrode layer
- 112: second electrode layer
- 113: third electrode layer
- 121: first insulating layer
- 122: second insulating layer
- 123: third insulating layer
- 130: component layer
- 140: power supply layer
- 210: positive electrode
- 211: positive electrode
- 212: positive electrode
- 220: negative electrode
- 222: insulating member
- 230: guard electrode
- 232: positive guard electrode
- 234: negative guard electrode
- 240: reference electrode
- 310: semiconductor element
- 311: wiring
- 312: electric field generating circuit
- 314: voltage detection circuit
- 316: A/D converter
- 318: potential adjustment circuit
- 320: first communication part
- 410: second communication part
- 420: controller
- 430: storage
- 440: prediction part
- 450: estimation part
- 460: output par
- 500: seat belt

## Claims

1. A detection device comprising:
a substrate; wherein
the substrate is provided with
a positive electrode and a negative electrode,
an electric field generating circuit that supplies a current or voltage between the positive electrode and the negative electrode to generate an electric field from a first side of the substrate,
a guard electrode that is located farther from the first surface than the positive electrode and the negative electrode, and that faces at least one of the positive electrode and the negative electrode via an insulator,
a potential adjustment circuit that makes i) the potential applied to the electrode corresponding to the guard electrode among the positive electrode and the negative electrode and ii) the potential of the guard electrode the same, and
a semiconductor element that outputs an electric field intensity detection value corresponding to the electric field intensity between the positive electrode and the negative electrode.

2. The detection device according to claim 1, wherein
the guard electrode includes a positive guard electrode facing the positive electrode and a negative guard electrode facing the negative electrode.

3. The detection device according to claim 1 or 2, wherein
the substrate includes
a first electrode layer provided with the positive electrode and the negative electrode,
a second electrode layer provided with the guard electrode,
a third electrode layer provided with a reference electrode providing a reference potential, and
the first electrode layer, the second electrode layer, and the third electrode layer are arranged in the order of the first electrode layer, the second electrode layer, and the third electrode layer from the first surface of the substrate to a surface opposite to the first surface.

4. The detection device according to claim 3, wherein
the substrate further includes a mounting part where at least one of an accelerometer, a gyroscope, and a six-axis sensor is mounted at a position farther from the first surface than the third electrode layer.

5. The detection device according to any one of claims 1 to 4, further comprising:
a power supply layer that supplies power to the electric field generating circuit, the potential adjustment circuit, and the semiconductor element.

6. The detection device according to any one of claims 1 to 5, further comprising:
a first communication part that generates a signal based on the detected electric field intensity value outputted from the semiconductor element and supplies the signal to the outside of the substrate.

7. A measuring system comprising:
the detection device according to any one of claims 1 to 6 that is arranged in a manner that the electric field generated from the positive electrode and the negative electrode reaches at least a portion of a living body; and
a measuring device that measures a state of the living body on the basis of the detection result of the detection device.

8. The measuring system according to claim 7, wherein
the measuring device includes
a second communication part that communicates with the detection device to receive the signal based on the electric field intensity detection value, and
an estimation part that estimates at least one of the movement, heartbeat, and blood pressure of the living body on the basis of the received signal.

9. The measuring system according to claim 8, wherein
the measuring device further includes a prediction part that predicts a future state of the living body on the basis of an estimation result of the estimation part.

10. The measuring system according to claim 9, wherein
the substrate is further provided with a temperature sensor,
the detection device transmits a signal based on a detection result of the temperature sensor and the electric field intensity detection value, and
the prediction part predicts at least one of a variation in the body temperature of the living body and a balance state of salt and water inside the living body, on the basis of the successive detection results of the temperature sensor.

11. The measuring system of according to any one of claims 7 to 10, further comprising:
a seat belt mounted on a seat; wherein
the detection device is mounted on the seat belt, and
the measuring device communicates with one or more of the detection devices to measure the state of the living body seated in the seat and fastening the seat belt.

12. The measuring system according to any one of claims 7 to 10, wherein
one or more of the detection devices is attached to clothing worn by the living body, and
the measuring device communicates with one or more of the detection devices to measure the state of the living body.

13. A monitoring system comprising:
the measuring system according to any one of claims 7 to 12;
acquisition parts that are connected to the measuring devices and acquire measurement results indicating the state of the living body; and
a detection part that detects an abnormality of the living body.

14. A program that, when executed by a computer, causes the computer to function as at least a part of the monitoring system according to claim 13.
